# EUROPÄISCHE PATENTSCHRIFT

(11) **EP 1 809 180 B1**
(45) Veröffentlichungstag und Bekanntmachung des Hinweises auf die Patenterteilung: **16.04.2008**
(21) Anmeldenummer: 05791410.3
(22) Anmeldetag: 18.10.2005
(51) Int. Cl.: A61B 17/00, A61B 17/08

(54) **SELBSTSCHLIESSENDER EXTERNER GEFÄSSVERSCHLUSS**
SELF-CLOSING EXTERNAL VESSEL CLOSURE
SYSTEME EXTERNE D'OBTURATION DE VAISSEAU SANGUIN A OBTURATION AUTOMATIQUE

(30) Priorität: 20.10.2004 CH 17312004
(43) Veröffentlichungstag der Anmeldung: 25.07.2007
(73) Patentinhaber: INOVA MEDICAL AG, 6330 Cham (CH)
(72) Erfinder: HARREN, Ernst-Diethelm, CH-6312 Steinhausen (CH); LEHMANN, Marc, Ulrich, CH-8704 Herrliberg (CH)
(74) Vertreter: EGLI-EUROPEAN PATENT ATTORNEYS
(86) Internationale Anmeldenummer: PCT/CH2005/000609
(87) Internationale Veröffentlichungsnummer: WO 2006/042431

(56) Entgegenhaltungen:
- WO-A-03/082127
- DE-A- 19 726 386

## Beschreibung

Die Erfindung betrifft einen selbstschliessenden externen Gefässverschluss zum Verschliessen eines eine Punktionsöffnung aufweisenden arteriellen oder venösen Blutgefässes im menschlichen oder tierischen Körper mittels Eigenblut gemäss Patentanspruch 1. Weitere Anwendungen sind auch bei mit Arterien kurzgeschlossenen Venen, bei Shunts, bei Prothesen und dergleichen.

Die Erfindung betrifft insbesondere einen derartigen Gefässverschluss mit einer mit Überdruck beaufschlagbaren, im Bereich der Punktionsöffnung am Körper befestigbaren Druckkammer, wobei diese Druckkammer bei einer Punktierung zum Zweck der Blutstillung mittels der Erzeugung eines Druckgleichgewichtes mit dem aus dem Blutgefäss ausströmenden Blut gefüllt wird.

Es besteht noch immer ein Bedarf, insbesondere in klinischen und polyklinischen Einrichtungen, einfach herstellbare, gut zu handhabende und zuverlässig wirkende selbstschliessende externe Gefässverschlüsse zum Verschliessen einer Punktionsöffnung mittels Eigenblut bereitzustellen.

Die EP-0 955 901 zeigt einen dichten Punktionsverschluss dieser Art in zwei Ausführungsformen:

Eine erste Ausführungsform gemäss EP-0 955 901 zeigt einen externen Punktionsverschluss zum Verschliessen eines eine Punktionsöffnung aufweisenden arteriellen Blutgefässes im menschlichen oder tierischen Körper mittels Eigenblut, mit einer mit Überdruck beaufschlagbaren, im Bereich der Punktionsöffnung am Körper befestigbaren Druckkammer, die in ihrem vom Körper abgewandten Bereich ein zweistückig ausgebildetes Verschlussteil, bestehend aus einer Haltewand aus polyetherurethan- Polyether- oder Polypropylenfolie und einem Verschlusselement aus Silikon, aufweist. Dabei können die Haltewand und das Verschlussteil miteinander verklebt sein. Die Druckkammer weist in ihrem dem Körper zugewandten Bereich ein einstückig ausgebildetes Druckwandteil auf.

Dieser externe Gefässverschluss ist aufklebbar, transparent und von einer Nadel oder Kanüle oder dergleichen durchstechbar.

Nachteile ergeben sich jedoch, weil die Haltewand und das Verschlussteil separat hergestellt und miteinander verbunden [verklebt oder verschweisst] werden müssen, was einen höheren Herstellungsaufwand bedingt. Allerdings ist vorgesehen, dass die Verbindung des Druckwandteiles mit der Haltewand durch Kleben mittels eines Silikonklebers oder, bei Materialgleichheit des Druckwandteiles und der Haltewand, auch durch Verschweissen erfolgen kann.

Eine zweite Ausführungsform gemäss EP-0 955 901 zeigt einen externen Gefässverschluss zum Verschliessen eines eine Punktionsöffnung aufweisenden arteriellen Blutgefässes im menschlichen oder tierischen Körper mittels Eigenblut, mit einer mit Überdruck beaufschlagbaren, im Bereich der Punktionsöffnung am Körper befestigbaren Druckkammer, die in ihrem vom Körper abgewandten Bereich ein einstückig ausgebildetes dickes Verschlussteil aus Silikon mit einem integrierten Verschlusselement aufweist. Die Druckkammer weist in ihrem dem Körper zugewandten Bereich ein einstückig ausgebildetes Druckwandteil auf.

Dieser externe Gefässverschluss ist ebenfalls aufklebbar, transparent und von einer Nadel oder Kanüle durchstechbar.

Nachteile ergeben sich jedoch, weil die Dicke des Verschlussteiles es verunmöglicht, dass sich der hier beschriebene arterielle Punktionsverschluss gut an die örtliche Hauttopographie anpassen kann. Ausserdem muss die vorgesehene Kleberschicht, die direkten Hautkontakt hat, auch gute hautverträgliche Eigenschaften aufweisen, was wiederum aber bei Klebstoffen, die für Silikon geeignet sind, nicht ohne weiteres zutrifft. Zudem besteht wegen der kleinflächigen Verklebung zwischen dem Verschlussteil und der Druckwand die Gefahr der Ablösung.

Insgesamt wird das Problem der Hautverträglichkeit von Silikonklebern bei keiner der zwei Ausführungsformen gemäss EP-0 955 901 berührt.

Aufgabe der Erfindung ist es, einen verbesserten und in der Herstellung besonders einfachen, selbstschliessenden externen Gefässverschluss anzugeben.

Diese Aufgabe wird durch die Merkmale des Patentanspruchs 1 gelöst.

Die Lösung besteht darin, dass bei einem gattungsgemässen selbstschliessenden externen Gefässverschluss mit einem einstückig ausgebildeten Verschlussteil aus Silikon mit einem integrierten Verschlusselement, und einem ebenfalls einstückig ausgebildeten Druckwandteil das Druckwandteil so ausgebildet ist, dass es sich mit Ausläufern auf den gesamten auf den auf den Körper aufzuklebenden Bereich ausdehnt, zudem das Druckwandteil ganz mit einer speziell hautverträglichen Hautkleberschicht versehen ist und zwischen dem Druckwandteil und dem Verschlussteil mit Ausnahme des Druckkammerbereiches grossflächig eine Kleberschicht angebracht ist. Die zuletzt erwähnte Kleberschicht braucht wegen fehlendem Hautkontakt keine speziell hautverträglichen Eigenschaften aufzuweisen und ist zudem im gesamten Einstichbereich ausgespart. Sie kann vielmehr so gewählt werden, dass optimale Klebeeigenschaften bezüglich der zu verbindenden Materialien resultieren, was im Fall von Silikon in der Regel spezielle Kleber und Vorbehandlungen erfordert.

Es kann auch vorgesehen sein, dass die Kleberschicht, die im einfachsten Fall nur aus einem Kleber (auch als technischer Kleber bezeichnet, d.h. ein Kleber ohne hautkleberspezifische Eigenschaften) besteht, einen Trägerfilm aufweist, der beidseitig mit Kleber versehen ist. Ein solcher Aufbau der Kleberschicht kann gewählt werden, um die Produktion in dem Sinne zu vereinfachen, dass die Kleberschicht auch als ,Zulieferteil' für die Herstellung des selbstschliessenden externen Gefässverschlusses konzipiert werden kann.

Das im Verschlussteil integrierte Verschlusselement ist dabei vorzugsweise kugelsegmentartig oder linsehartig, rund oder elliptisch ausgebildet. Das Verschlussteil ist vorzugsweise im Bereich des integrierten Verschlusselementes mindestens 4 mm dick. Dadurch lässt sich der externe Gefässverschluss besonders leicht mit Überdruck beaufschlagen.

Indem der selbstschliessende externe Gefässverschluss mit einer Anzahl von weichen und dünnen Ausläufern versehen wird, erreicht man eine gute und sichere Befestigung des externen Gefässverschlusses an unterschiedlichste Hauttopographien. Da der erfindungsgemässe selbstschliessende externe Gefässverschluss auch in den Ausläufern aus nur zwei Materialschichten besteht, nämlich aus einer oberen, aus dem einstückig ausgebildeten Verschlussteil ausgeformten Schicht, und einer unteren, aus dem ebenfalls einstückig ausgebildeten Druckwandteil ausgeformten Schicht, erreicht man optimale Anpassungsmöglichkeiten bezüglich der mechanischen Anforderungen, und zwar in mehrfacher Hinsicht. Da ist einerseits die sichere und hautverträgliche Haftung des externen Gefässverschlusses auf der Haut, andererseits die sichere und zuverlässige Haftung des aus Silikon bestehenden Verschlussteiles auf dem Druckwandteil. Da das Verschlussteil im Bereich des integrierten kugelsegmentartigen Verschlusselementes dort auch die Funktion einer Stabilisierungswand zum Zweck des Aufbaus eines Gegendruckes übernimmt, muss nämlich auch sichergestellt bleiben, dass die durch das ausströmende Blut sich füllende Druckkammer sich in Richtung des körperinneren ausbildet. Deshalb kommt auch in den Übergangsbereichen zwischen den Ausläufern und der Druckkammer der Zuverlässigkeit der Klebung und der Materialkombination eine ganz besondere Bedeutung bei. Dies um so mehr, als Silikon zwar die gewünschten Wiederverschliesseigenschaften nach dem Durchstechen mit einer Kanüle oder Nadel aufweist, gleichzeitig aber auch eine aussergewöhnlich hohe Dehnfähigkeit hat. Erfindungsgemäss wird also mit der resultierenden Wahlfreiheit für einen wirklich zuverlässigen Silikonkleber erreicht, dass sich die Klebung in den Übergangsbereichen bei den stossartig auftretenden Druckspitzen nicht löst. Um die Klebung weiter zu verbessern, kann auch vorgesehen sein, das Silikon des Verschlussteils kleberschichtseitig vor der Klebung einer Plasma-, corona-, nasschemischen oder sonstigen Behandlung zu unterziehen.

Hinsichtlich der Materialkombination zwischen dem Verschlussteil aus Silikon und dem Druckwandteil erweist es sich als vorteilhaft, für letzteres ebenfalls ein weiches, sich der Hauttopographie gut anpassenden Material von hoher, in der Regel aber geringerer Dehnfähigkeit als Silikon, vorzusehen. Das Material für den Druckwandteil muss in dem der Punktionsöffnung zugewandten Druckkammerbereich eine dehnbare Druckwand ausbilden können. Vorzugsweise ist das Druckwandteil aus einer 5 µm bis 50 µm dicken, vorzugsweise aber ca. 25 µm dicken Polyetherurethan-, Polyether oder Polypropylenfolie ausgebildet.

Vorzugsweise wählt man auch eine Hautkleberschicht, die durch Beigabe entsprechender Wirkstoffe eine oder mehrere der Eigenschaften aus der folgenden Gruppe von Eigenschaften aufweist:
- antiseptische Eigenschaften,
- antiallergische Eigenschaften,
- analgetische Eigenschaften.

Im folgenden wird der erfindungsgemässe selbstschliessende externe Gefässverschluss anhand eines Ausführungsbeispieles mit Zeichnungen detailliert beschrieben.

In den Zeichnungen zeigen:
- Fig. 1: einen Querschnitt durch einen selbstschliessenden externen Gefässverschluss, im Zustand vor der Punktierung eines Blutgefässes,
- Fig. 2: einen weiteren Querschnitt durch einen selbstschliessenden externen Gefässverschluss gemäss Fig. 1, im Zustand nach Abschluss der Punktierung eines Blutgefässes, und
- Fig. 3: Eine Draufsicht auf einen selbstschliessenden externen Gefässverschluss gemäss Fig. 1.

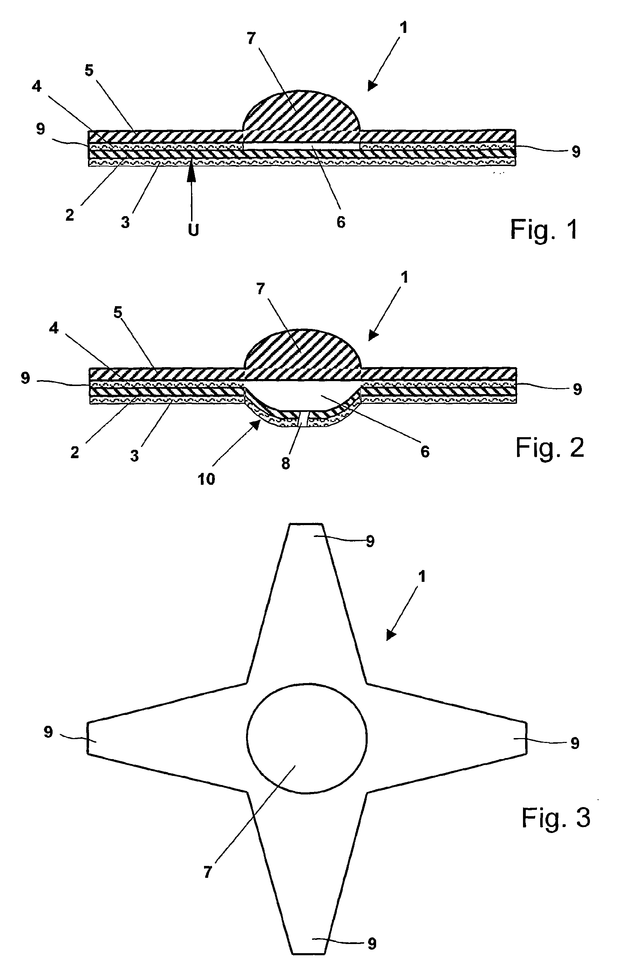

Die Figur 1 zeigt einen nicht massstäblichen Querschnitt durch einen selbstschliessenden externen Gefässverschluss (1), wobei der Gefässverschluss (1) im Zustand vor der Punktierung eines Blutgefässes dargestellt ist.

Ein in der Regel dünnes (folienartiges) einstückiges Druckwandteil 2 ist beidseitig mit Klebstoffschichten versehen. An einer Unterseite U des Druckwandteiles 2 befindet sich eine (medizinische) Hautkleberschicht 3, wobei diese Seite des selbstschliessenden externen Gefässverschluss 1 zum Aufkleben auf die menschliche Haut vorgesehen ist. Auf der gegenüberliegenden Seite des Druckwandteiles 2 befindet sich eine (technische) Kleberschicht 4. Die Kleberschicht 4 befindet sich zwischen dem Druckwandteil 2 und einem ebenfalls einstückig ausgebildeten Verschlussteil 5 und hat keine hautkleberspezifischen Eigenschaften. Das Verschlussteil 5 ist vorzugsweise aus Silikon. Silikon ist ein Material, für das derzeit allerdings nur Kleber bekannt sind, die keine oder keine besonders gute Hautverträglichkeit aufweisen. Beim vorgesehenen Aufbau des selbstschliessenden externen Gefässverschlusses 1 braucht die Kleberschicht 4 keine hautkleberspezifischen bzw. besonders hautfreundlichen Eigenschaften aufzuweisen, da sie keinen Hautkontakt hat. Die Kleberschicht 4 hat ausserdem auch keinen Kontakt mit einer Kanüle, Nadel oder dergleichen hat, da die Kleberschicht 4 im Einstichbereich ausgespart ist.

Der selbstschliessende externe Gefässverschluss 1 hat eine mit Überdruck beaufschlagbare, im Bereich der Punktionsöffnung am Körper befestigbare Druckkammer 6. Die Druckkammer 6 liegt in einem Zwischenraum zwischen dem Verschlussteil 5 und dem Druckwandteil 2, bzw. sie kann sich in diesem Zwischenraum ausbilden. Die Druckkammer 6 weist in ihrem vom Körper abgewandten Bereich, also am einstückigen Verschlussteil 5, ein im Verschlussteil integriertes, kugelsegmentartiges oder linsenartiges, rundes oder elliptisches Verschlusselement 7 auf. Die Druckkammer 6 besteht in ihrem dem Körper zugewandten Teil lediglich aus einem mit dem Verschlussteil 5 nicht verklebten Teilstück des dünnen einstückigen Druckwandteils 2. Die Druckkammer kann auch mit einem Gerinnungsmittel bzw. einem Hämostatikum versehen sein.

Das Verschlusselement 7 dient dazu, die Druckkammer 6 des selbstschliessenden externen Gefässverschlusses 1 von aussen mit einem Überdruck beaufschlagen zu können. Das Verschlussteil 5, bzw. dessen verdickter Bereich des integrierten kugelsegmentartigen Verschlusselementes 7, übernimmt bei der Druckkammer 6 aber auch die Funktion einer Stabilisierungswand zum Zweck des Aufbaus eines Gegendruckes, indem es sicherstellt, dass die durch das ausströmende Blut sich füllende Druckkammer 6 sich stets in Richtung des Körperinneren ausbildet.

Alle verwendeten Schichten und Teile, d.h. das Druckwandteil 2, das Verschlussteil 5, die Kleberschicht 4 und die Hautkleberschicht 3 sind vorzugsweise im ganzen Bereich des selbstschliessenden externen Gefässverschlusses 1 oder aber zumindest in einem genügend grossen übereinanderliegenden Teilbereich desselben transparent oder annähernd transparent. Zudem sind für das Druckwandteil 2 und das Verschlussteil 5 weiche und sich der Hauttopographie gut anpassende Materialien vorgesehen. Das Druckwandteil 2 ist zudem vorzugsweise aus einem Material, das weniger dehnbar ist als das Verschlussteil 5. Damit wird die höhere Dehnfähigkeit der Verschlussschicht 5, insbesondere im Fall von Silikon, in der flächigen Ausdehnung begrenzt und stabilisiert.

Wird für die Kleberschicht 4 ein Schichtaufbau mit einem Trägerfilm und beidseitig auf dem Trägerfilm aufgebrachtem (technischen) Kleber verwendet, so ist natürlich zu erwarten, dass dieser Trägerfilm, ebenso wie das Druckwandteil 2, und natürlich auch in Abhängigkeit von der Materialwahl, zur Ausdehnungsbegrenzung und Stabilisierung des Verschlussteils 5 beiträgt.

Eine besonders günstige und bevorzugte Materialkombination besteht darin, dass das Verschlussteil 5 aus Silikon und das Druckwandteil 2 aus einer 5 µm bis 50 µm, vorzugsweise aber ca. 25 µm, dicken Polyetherurethan-, Polyether oder Polypropylenfolie ausgebildet ist. Vorteilhaft ist das Verschlussteil 5 im Bereich des integrierten Verschlusselementes 7 auch mindestens etwa 4 mm dick.

Die Figur 2 zeigt einen weiteren Querschnitt durch einen selbstschliessenden externen Gefässverschluss gemäss Fig. 1, hier jedoch im Zustand nach Abschluss der Punktierung eines Blutgefässes. Die Druckkammer 6 ist hier mit Blut gefüllt (nicht dargestellt). Die dem Körper zugewandte Seite der Druckkammer 6 weist eine Durchstichöffnung 8 auf, durch die das Blut in die Druckkammer 6 einströmen konnte. Die dem Körper abgewandte Seite der Druckkammer 6, bzw. das Verschlusselement 7, wurde zwar ebenfalls durchstochen, deren Einstichkanal hat sich aber dank der ausserordentlich hohen Rückstellkraft von Silikon nach dem Herausziehen der Nadel oder Kanüle von selbst wieder verschlossen. Somit konnte sich die in Richtung des Körperinneren ausgewölbte und dargestellte Form der Druckkammer 6 ausbilden.

Die Figur 3 zeigt schliesslich noch eine Draufsicht auf einen selbstschliessenden externen Gefässverschluss gemäss Fig. 1. Vorteilhafterweise ist der selbstschliessende externe Gefässverschluss 1 mit einer Anzahl von weichen und dünnen Ausläufern 9 versehen, womit man eine gute und sichere Befestigung des selbstschliessenden externen Gefässverschlusses an unterschiedlichsten Hauttopographien erreicht.

Der erfindungsgemässe selbstschliessende externe Gefässverschluss 1 besteht somit mit Ausnahme des eigentlichen Druckkammerbereiches (Druckwand 10), also im wesentlichen im Bereich der Ausläufer 9, in der Regel aus nur zwei mittels der (technischen) Kleberschicht 4 grossflächig miteinander verklebten Materialschichten, nämlich aus der oberen und dickeren Materialschicht aus dem Material des einstückig ausgebildeten Verschlussteils 5 mit dem integriertem Verschlusselement 7 und der unteren und dünneren Materialschicht aus dem Material des ebenfalls einstückig ausgebildeten Druckwandteils 2 mit der hautseitig aufgebrachten Hautkleberschicht 3.

Es kann allerdings auch vorgesehen sein, die Hautkleberschicht 3 mittels einer in der Regel ebenfalls dünnen (folienartigen) Trägerschicht (nicht dargestellt) auf das Druckwandteil 2 aufzubringen. Bei dieser Ausführungsart ist zwischen dem Druckwandteil 2 und der Trägerschicht eine weitere (technische) Kleberschicht vorhanden (ebenfalls nicht dargestellt). Diese weitere (technische) Kleberschicht kann sich somit ohne weiteres auf die gesamte Unterseite des selbstschliessenden externen Gefässverschlusses erstrecken. Bei dieser Ausführungsart ist die Trägerschicht vorzugsweise ebenfalls aus einem Material, das weniger dehnbar ist als das Verschlussteil 5. Damit wird die höhere Dehnfähigkeit der Verschlussschicht 5, insbesondere im Fall von Silikon, in der flächigen Ausdehnung weiter begrenzt und stabilisiert.

Bei der zuletzt erwähnten Ausführungsform kann für die Trägerschicht auch ein absorptionsfähiges Material wie beispielsweise Vliessstoff oder Papier eingesetzt werden.

Bei allen Ausführungsvarianten kann zudem vorgesehen sein, dass wasserdampfdurchlässige Materialien verwendet werden.

Die Figur 3 zeigt beispielhaft eine Ausführungsform mit vier Ausläufern 9. Es kann natürlich auch eine andere geeignete Zahl von Ausläufern 9 vorgesehen sein. So könnte die Zahl der Ausläufer bis zu einer insgesamt flächigen und ausläuferlosen Form 'vervielfacht' werden. Letzteres würde dann aber das sichere Aufbringen an topographisch ungünstigen Körperstellen eher erschweren. Die Länge der Ausläufer 9 hat natürlich auch einen wesentlichen Einfluss auf die Haftfähigkeit des selbstschliessenden externen Gefässverschlusses 1 auf der Haut und muss demzufolge natürlich dem Anwendungszweck entsprechend gewählt oder konfektioniert werden.

Zur Anwendung des selbstschliessenden externen Gefässverschlusses:
- Das zur Punktierung vorgesehene Blutgefäss wird lokalisiert.
- Die zu punktierende Körperstelle wird in herkömmlicher Weise gereinigt und desinfiziert.
- Beim selbstschliessenden externen Gefässverschluss 1 werden die Schutzfolien abgezogen und der Gefässverschluss 1 wird mit der Hautkleberschicht 3 auf die betreffende Körperstelle aufgeklebt. Damit wird der vor der Punktierung geschaffene antiseptische Zustand vor der Punktierung konserviert.
- Im Falle des zur Punktierung vorgesehenen Gefässes wird dasselbe visuell durch die transparente Oberfläche des selbstschliessenden externen Gefässverschlusses erneut lokalisiert.
- Die Punktierung wird durchgeführt, indem der externe Gefässverschluss bzw. alle Schichten desselben (mit Ausnahme der Kleberschicht 4, die im Punktierungsbereich ausgespart ist) im Bereich des Verschlusselementes 7 mit einer Nadel oder Kanüle durchstochen. In besonderen Fällen wird bei diagnostischen Eingriffen mittels eines Katheter durchstochen. Dem zu behandelnden Patienten wird die vorgesehene Menge Flüssigkeit durch die Nadel oder Kanüle entnommen und/oder zugeführt oder aber die diagnostische Massnahme durchgeführt. Da stets davon ausgegangen wird, dass der Eingriff von medizinischem Fachpersonal durchgeführt wird, wird auch vorausgesetzt, dass die verwendete Spritze oder Kanüle oder Katheter oder sonstiges Gerät bereits steril ist. Der antiseptische Zustand wird somit auch während der Punktierung erhalten. Wegen der elastischen Rückstellkraft der Verschlussschicht 5 schützt dieselbe die zu punktierende Stelle auch während des Eingriffes vor Umwelteinflüssen, weil das Material stets dicht an der Nadel oder Kanüle anliegt. Zudem wird das Personal vor möglichen Infektionsrisiken geschützt.
- Nach erfolgtem Eingriff wird die Nadel oder Kanüle oder Katheter oder dergleichen herausgezogen. Abermals wirkt die elastische Rückstellkraft des Verschlussteils 5 in einer Weise, dass der Einstichkanal im Verschlusselement 7 durch die Rückstellkraft des Materials sofort wieder verschlossen wird. Somit wird die punktierte Stelle auch nach dem Eingriff zuverlässig geschützt. Es können keine Keime, mikroskopisch und/oder makroskopische Schmutzpartikel und/oder Viren und/oder Bakterien in den Einstichkanal und somit in den menschlichen oder tierischen Körper gelangen. Da nach dem Zurückziehen der Nadel oder Kanüle oder dergleichen die Durchstichöffnung 8 in der Druckwand 10 jedoch offen bleibt, kann daraufhin austretendes Blut durch den Einstichkanal und die Durchstichöffnung 8 in die Druckkammer 6 einfliessen und dieselbe auffüllen. Infolge der Stabilisierungswirkung des Verschlusselementes 7 formt sich die Druckkammer 6 (wie in der Figur 2 illustriert) in Richtung des Körperinneren aus. Sobald der Blutdruck in der Druckkammer 6 dem Druck des aus dem Einstichkanal austretenden Blutes entspricht, kommt die Blutung zum Stillstand.
- Durch die beschriebene Bauweise des selbstschliessenden externen Gefässverschlusses wird der über die Füllung der Druckkammer hinausgehende Blutaustritt aus der Einstichwunde im Normalfall ausgeschlossen. Der externe Gefässverschluss kann jedenfalls so lange wie notwendig oder wünschenswert auf der betreffenden Körperstelle verbleiben.

### Bezugsziffernliste:

- 1: externer Gefässverschluss
- 2: Druckwandteil
- 3: (medizinische) Hautkleberschicht
- 4: (technische) Kleberschicht
- 5: Verschlussteil
- 6: Druckkammer
- 7: Verschlusselement
- 8: Durchstichöffnung
- 9: Ausläufer
- 10: Druckwand

- U: Unterseite des Druckwandteiles

## Patentansprüche

1. Selbstschliessender externer Gefässverschluss (1) zum Verschliessen eines eine Punktionsöffnung aufweisenden arteriellen oder venösen Blutgefässes im menschlichen oder tierischen Körper mittels Eigenblut, mit
- einer mit Überdruck beaufschlagbaren, im Bereich der Punktionsöffnung am Körper befestigbaren Druckkammer (6), die in ihrem vom Körper abgewandten Bereich ein einstückig ausgebildetes Verschlussteil (5) aus Silikon mit einem integrierten Verschlusselement (7) aufweist, und wobei die Druckkammer (6) in ihrem dem Körper zugewandten Bereich ein einstückig ausgebildetes Druckwandteil (2) aufweist, und
wobei der externe Gefässverschluss (1) aufklebbar, transparent und von einer Nadel einer Kanüle, einem Katheter oder dergleichen durchstechbar ist,
**dadurch gekennzeichnet, dass**
- das Druckwandteil (2) sich mit Ausläufern (9) auf den gesamten auf den Körper aufzuklebenden Bereich des externen Gefässverschlusses (1) erstreckt , und
- zwischen dem Druckwandteil (2) und dem Verschlussteil (5) mit Ausnahme des Druckkammerbereiches grossflächig eine Kleberschicht (4) angebracht ist.

2. Selbstschliessender externer Gefässverschluss (1) nach Patentanspruch 1, **dadurch gekennzeichnet, dass** das integrierte Verschlusselement (7) kugelsegmentartig oder linsenartig, rund oder elliptisch ausgebildet ist.

3. Selbstschliessender externer Gefässverschluss (1) nach Patentanspruch 1 oder 2, **dadurch gekennzeichnet, dass** sich das Verschlussteil (5) auf die Ausläufer (9) erstreckt, wobei die Ausläufer (9) in diesem Bereich mindestens 1 mm dick sind.

4. Selbstschliessender externer Gefässverschluss (1) nach einem der Patentansprüche 1 bis 3, **dadurch gekennzeichnet, dass** das Verschlussteil (5) im Bereich des integrierten Verschlusselementes (7) mindestens 4 mm dick ist.

5. Selbstschliessender externer Gefässverschluss (1) nach einem der Patentansprüche 1 bis 4, **dadurch gekennzeichnet, dass** das Druckwandteil (2) aus einem weichen, sich der Hauttopographie gut anpassenden Material von hoher, aber geringerer Dehnfähigkeit als Silikon ausgebildet ist und insbesondere in ihrem der Punktionsöffnung zugewandten Druckkammerbereich eine dehnbare Druckwand (10) aufweist.

6. Selbstschliessender externer Gefässverschluss (1) nach Patentanspruch 5, **dadurch gekennzeichnet, dass** das Druckwandteil (2) aus einer 5 µm bis 50 µm, vorzugsweise 25 µm dicken Polyetherurethan-, Polyether oder Polypropylenfolie ausgebildet ist.

7. Selbstschliessender externer Gefässverschluss (1) nach einem der Patentansprüche 1 bis 6, **dadurch gekennzeichnet, dass** die Kleberschicht (4) keine hautkleberspezifischen Eigenschaften aufzuweisen braucht.

8. Selbstschliessender externer Gefässverschluss (1) nach einem der Patentansprüche 1 bis 7, **dadurch gekennzeichnet, dass** die Kleberschicht (4) einen Trägerfilm aufweist der beidseitig mit Kleber versehen ist.

9. Selbstschliessender externer Gefässverschluss (1) nach einem der Patentansprüche 1 bis 8, **dadurch gekennzeichnet, dass** eine Hautkleberschicht (3) vorhanden ist, die durch beigegebene Wirkstoffe eine oder mehrere der Eigenschaften aus der folgenden Gruppe von Eigenschaften aufweist:
- antiseptische Eigenschaften,
- antiallergische Eigenschaften,
- analgetische Eigenschaften.

10. Selbstschliessender externer Gefässverschluss (1) nach Patentanspruch 9, **dadurch gekennzeichnet, dass** die Hautkleberschicht (3) auf einer Unterseite U auf dem Druckwandteil (2) oder mittels einer Trägerschicht und einer weiteren Kleberschicht auf der Unterseite U des Druckwandteils aufgebracht ist.

11. Selbstschliessender externer Gefässverschluss (1) nach Patentanspruch 10, **dadurch gekennzeichnet, dass** die Trägerschicht flüssigkeitsabsorbierend ist.

12. Selbstschliessender externer Gefässverschluss (1) nach einem der Patentansprüche 9 bis 11, **dadurch gekennzeichnet, dass** die Hautkleberschicht 3 von einer abziehbaren Schutzfolie bedeckt ist.

13. Selbstschliessender externer Gefässverschluss (1) nach einem der Patentansprüche 1 bis 12, **dadurch gekennzeichnet, dass** das Verschlussteil (5) vorzugsweise aus kleberschichtseitig plasma-, corona-, nasschemisch- oder sonstig vorbehandeltem Silikon ist.

14. Selbstschliessender externer Gefässverschluss (1) nach einem der Patentansprüche 1 bis 13, **dadurch gekennzeichnet, dass** wasserdampfdurchlässige Materialien verwendet werden.

15. Selbstschliessender externer Gefässverschluss (1) nach einem der Patentansprüche 1 bis 14, **dadurch gekennzeichnet, dass** die Druckkammer 6 mit einem Gerinnungsmittel versehen ist.

## Claims

1. Self-closing external vessel closure (1) for closing a punctured arterial or venous blood vessel in the human or animal body by means of the human's or animal's own blood, comprising
- a pressure chamber (6) which can be impinged upon by excess pressure, which can be attached to the body in the area of the puncture and which is provided with a monolithically embodied silicone closing part (5) with an integrated closing element (7) in its zone facing away from the body, and wherein the pressure chamber (6) is fitted with a monolithically embodied pressure wall part (2) in its area facing the body, and
wherein the external vessel closure (1) is adhesive, transparent and can be pricked by a needle of a cannula, a catheter or similar,
**characterised in that**
- the pressure wall part (2) extends with extensions (9) to the entire area of the external vessel closure (1) that can be affixed to the body and
- an adhesive layer (4) is mounted on a large area between the pressure wall part (2) and the closing part (5) with the exception of the region of the pressure chamber.

2. The self-closing external vessel closure (1) according to claim 1, **characterised in that** the integrated closing element (7) is configured as spherical-segment-like or lens-like, round or elliptical.

3. The self-closing external vessel closure (1) according to claim 1 or 2, **characterised in that** the closing part (5) extends onto the extensions (9), wherein the extensions (9) are at least 1 mm thick in this region.

4. The self-closing external vessel closure (1) according to any one of claims 1 to 3, **characterised in that** the closing part (5) is at least 4 mm thick in the area of the integrated closing element (7).

5. The self-closing external vessel closure (1) according to any one of claims 1 to 4, **characterised in that** the pressure wall part (2) is formed from a soft material which is well suited to the skin topography and has a high expandability but lower than that of silicone, and in particular has an expandable pressure wall (10) in the region of its pressure chamber facing the puncture.

6. The self-closing external vessel closure (1) according to claim 5, **characterised in that** the pressure wall part (2) is formed from 5 µm to 50 µm, preferably 25 µm thick polyurethane, polyether or polypropylene film.

7. The self-closing external vessel closure (1) according to any one of claims 1 to 6, **characterised in that** the adhesive layer (4) does not need to have any skin-adhesive-specific properties.

8. The self-closing external vessel closure (1) according to any one of claims 1 to 7, **characterised in that** the adhesive layer (4) comprises a supporting film which is provided with adhesive on both sides.

9. The self-closing external vessel closure (1) according to any one of claims 1 to 8, **characterised in that** a skin adhesive layer (3) is provided which exhibits one or more properties from the following group of properties as a result of added active substances:
- antiseptic properties,
- anti-allergic properties,
- analgesic properties.

10. The self-closing external vessel closure (1) according to claim 9, **characterised in that** the skin adhesive layer (3) is applied to a bottom face U on the pressure wall part (2) or to the bottom face U of the pressure wall part by means of a supporting layer and a further adhesive layer.

11. The self-closing external vessel closure (1) according to claim 10, **characterised in that** the supporting layer is liquid-absorbing.

12. The self-closing external vessel closure (1) according to any one of claims 9 to 11, **characterised in that** the skin adhesive layer (3) is covered with a peelable protective film.

13. The self-closing external vessel closure (1) according to any one of claims 1 to 12, **characterised in that** the closing part (5) is preferably made of silicone which has undergone plasma, corona, wet-chemical or other pretreatment on the adhesive layer side.

14. The self-closing external vessel closure (1) according to any one of claims 1 to 13, **characterised in that** water-vapour-permeable materials are used.

15. The self-closing external vessel closure (1) according to any one of claims 1 to 14, **characterised in that** the pressure chamber (6) is provided with a coagulant.

## Revendications

1. Fermeture externe autoverrouillante de vaisseau (1) pour fermer un vaisseau sanguin artériel ou veineux comportant un orifice de ponction dans le corps humain ou animal au moyen du propre sang, avec
- une chambre de compression (6), susceptible d'être soumise à une surpression, et d'être fixée dans la région de l'orifice de ponction sur le corps, qui dans sa région opposée au corps comporte une pièce de fermeture (5) en silicone conçue en monobloc avec un élément de fermeture (7) intégré, et la chambre de compression (6) comportant dans sa région dirigée vers le corps une pièce de paroi contre-pression (2) conçue en monobloc, et
la fermeture de vaisseau externe (1) étant susceptible d'être collée, transparente et transperçable par une aiguille ou par une canule, un cathéter ou similaire, **caractérisée en ce que**
- la pièce de paroi contre-pression (2) s'étend avec des extensions (9) sur l'ensemble de la région à coller sur le corps de la fermeture de vaisseau externe (1), et
- entre la pièce de paroi contre-pression (2) et la pièce de fermeture (5), une couche d'adhésif (4) est appliquée, en exceptant la région de la chambre de compression.

2. Fermeture externe autoverrouillante de vaisseau (1) selon la revendication 1, **caractérisée en ce que** l'élément de fermeture (7) intégré est conçu sous la forme d'un segment sphérique, d'une lentille, sous forme ronde ou elliptique.

3. Fermeture externe autoverrouillante de vaisseau (1) selon la revendication 1 ou 2, **caractérisée en ce que** la pièce de fermeture (5) s'étend sur les extensions (9), les extensions (9) ayant une épaisseur d'au moins 1 mm dans cette région.

4. Fermeture externe autoverrouillante de vaisseau (1) selon l'une quelconque des revendications 1 à 3, **caractérisée en ce que** la pièce de fermeture (5) a une épaisseur d'au moins 4 mm, dans la région de l'élément de fermeture (7) intégré.

5. Fermeture externe autoverrouillante de vaisseau (1) selon l'une quelconque des revendications 1 à 4, **caractérisée en ce que** la pièce de paroi contre-pression (2) est conçue dans une matière souple, s'adaptant bien à la topographie de la peau, bien extensible, mais moins que de la silicone et **en ce qu'**elle comporte notamment dans sa région de la chambre de compression dirigée vers l'orifice de ponction une paroi contre-pression (10) extensible.

6. Fermeture externe autoverrouillante de vaisseau (1) selon la revendication 5, **caractérisée en ce que** la pièce de paroi contre-pression (2) est conçue dans un film de polyuréthane, de polyéther ou de polypropylène d'une épaisseur de 5 µm à 50 µm, de préférence de 25 µm.

7. Fermeture externe autoverrouillante de vaisseau (1) selon l'une quelconque des revendications 1 à 6, **caractérisée en ce que** la couche d'adhésif (4) n'a pas besoin de présenter des propriétés spécifiques à un adhésif cutané.

8. Fermeture externe autoverrouillante de vaisseau (1) selon l'une quelconque des revendications 1 à 7, **caractérisée en ce que** la couche d'adhésif (4) comporte un film support muni sur ses deux faces d'adhésif.

9. Fermeture externe autoverrouillante de vaisseau (1) selon l'une quelconque des revendications 1 à 8, **caractérisée par** la présence d'une couche d'adhésif cutané (3), qui grâce à des principes actifs ajoutés présente l'une ou plusieurs des propriétés du groupe suivant de propriétés :
- propriétés antiseptiques,
- propriétés antiallergiques,
- propriétés analgésiques.

10. Fermeture externe autoverrouillante de vaisseau (1) selon la revendication 9, **caractérisée en ce que** la couche d'adhésif cutané (3) est appliquée sur une face inférieure U sur la pièce de paroi contre-pression (2) ou au moyen d'une couche support et d'une couche d'adhésif supplémentaire sur la face inférieure U de la pièce de paroi contre-pression.

11. Fermeture externe autoverrouillante de vaisseau (1) selon la revendication 10, **caractérisée en ce que** la couche support absorbe les liquides.

12. Fermeture externe autoverrouillante de vaisseau (1) selon l'une quelconque des revendications 9 à 11, **caractérisée en ce que** la couche d'adhésif cutané (3) est recouverte d'un film de protection pelliculable.

13. Fermeture externe autoverrouillante de vaisseau (1) selon l'une quelconque des revendications 1 à 12, **caractérisée en ce que** la pièce de fermeture (5) est de préférence en silicone prétraitée au plasma, corona, par traitement chimique humide ou par un autre traitement, côté couche d'adhésif.

14. Fermeture externe autoverrouillante de vaisseau (1) selon l'une quelconque des revendications 1 à 13, **caractérisée en ce qu'**on utilise des matières perméables à la vapeur d'eau.

15. Fermeture externe autoverrouillante de vaisseau (1) selon l'une quelconque des revendications 1 à 14, **caractérisée en ce que** la chambre de compression 6 est munie d'un agent coagulant.
